(19) Europäisches Patentamt
European Patent Office
Office européen des brevets

(11) EP 1 173 150 B1

(12) # EUROPÄISCHE PATENTSCHRIFT

(45) Veröffentlichungstag und Bekanntmachung des Hinweises auf die Patenterteilung:
**16.06.2004 Patentblatt 2004/25**

(21) Anmeldenummer: **00922608.5**

(22) Anmeldetag: **05.04.2000**

(51) Int Cl.$^7$: **A61K 7/50**

(86) Internationale Anmeldenummer:
**PCT/EP2000/003013**

(87) Internationale Veröffentlichungsnummer:
**WO 2000/061102 (19.10.2000 Gazette 2000/42)**

(54) **KOSMETISCHE UND/ODER PHARMAZEUTISCHE ZUBEREITUNGEN**

COSMETIC AND/OR PHARMACEUTICAL PREPARATIONS

PREPARATIONS COSMETIQUES ET/OU PHARMACEUTIQUES

(84) Benannte Vertragsstaaten:
**DE ES FR GB IT**

(30) Priorität: **10.04.1999 DE 19916211**

(43) Veröffentlichungstag der Anmeldung:
**23.01.2002 Patentblatt 2002/04**

(73) Patentinhaber: **Cognis Deutschland GmbH & Co. KG**
**40589 Düsseldorf (DE)**

(72) Erfinder:
- **SCHMID, Karl, Heinz**
  **D-40822 Mettmann (DE)**
- **FABRY, Bernd**
  **D-41352 Korschenbroich (DE)**
- **HENSEN, Hermann**
  **D-42781 Haan (DE)**
- **KOESTER, Josef**
  **D-40221 Düsseldorf (DE)**

(56) Entgegenhaltungen:
**EP-A- 0 258 814**
**WO-A-97/18033**
**DE-A- 4 012 693**
**DE-A- 4 108 626**
**DE-A- 19 749 819**
**FR-A- 2 785 794**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 173 150 B1

## Beschreibung

### Gebiet der Erfindung

Die Erfindung befindet sich auf dem Gebiet der Kosmetik und betrifft Zubereitungen, die spezielle Ester von Hydroxycarbonsäuren zusammen mit Fettalkoholen enthalten, sowie die Verwendung der Mischungen zur Herstellung von kosmetischen bzw. pharmazeutischen Zubereitungen.

### Stand der Technik

Aus der Europäischen Patentschrift **EP 0553241 B1** (SEPPIC) ist die Verwendung von Mischungen aus 10 bis 40 Gew.-% Alkyloligoglucosiden, 60 bis 90 Gew.-% Fettalkoholen und gegebenenfalls Polyglucose zur Herstellung von Emulsionen bekannt. Auch gemäß der Lehre der internationalen Patentanmeldung **WO 92/07543** (Henkel) lassen sich Alkyloligoglucoside zusammen mit Fettalkoholen und Partialglyceriden als kosmetische Emulgatoren einsetzen. Gegenstand der deutschen Patentschrift **DE 19542572 C2** (Henkel) ist die Verwendung von Emulgatoren, die 50 bis 90 Gew.-% Alkylglucoside und 10 bis 50 Gew.-% Fettalkohole enthalten.

Es zeigt sich jedoch, daß insbesondere zur Herstellung von hochviskosen Cremes die Glucosid/Fettalkohol-Emulgatoren des Stands der Technik in retativ hohen Mengen eingesetzt werden müssen. Hohe Wachskonzentrationen, d.h. hohe Mengen der Emulgatormischung, führen aber zu einer unerwünschten Belastung der Haut, insbesondere dann, wenn sensorisch leichte Produkte gewünscht werden. Ein weiteres Problem besteht darin, daß die Mischungen bei Temperaturbelastung innerhalb vergleichsweise kurzer Lagerung die Tendenz zeigen, sich zu entmischen. Es liegt auf der Hand, daß eine Verbraucherin ein Produkt, weiches sich bei Sonneneinstrahlung irreversibel in eine Fett- und eine Wasserphase trennt, kein zweitesmal kaufen wird.

Die Aufgabe der Erfindung hat somit darin bestanden, Emulgatoren vorzugsweise für die Herstellung hochviskoser Emulsionen zur Verfügung zu stellen, die gegenüber den Produkten des Stands der Technik die Herstellung stabiler, aber gleichzeitig sensorisch leichter Produkte mit einer verminderten Menge an Wachskörpem erlauben.

### Beschreibung der Erfindung

Gegenstand der Erfindung sind kosmetische Zubereitungen, enthaltend

(a) Hydroxycarbonsäurealkyl- und/oder -alkenyloligoglykosidester und
(b) Fettalkohole.

Überraschenderweise wurde gefunden, daß Mischungen der speziellen Hydroxycarbonsäureester mit Fettalkoholen nicht nur über selbstemulgierende Eigenschaften verfügen und Emulsionen auch bei längerer Lagerung und Temperaturbelastung eine verbesserte Stabilität verleihen, sondern insbesondere auch eine synergistische Verbesserung der verdickenden Eigenschaften bewirken. Auf diese Weise können definierte Viskositäten mit vergieichsweise geringeren Konzentrationen an Verdickungsmittel eingestellt werden. Gleichzeitig wird auf diesem Wege die Herstellung sensorisch leichter Produkte ermöglicht.

Hydroxycarbonsäurealkyl- und/oder -alkenyloligoglykosidester

Ester von Hydroxycarbonsäuren und Alkyl- und Alkenyloligoglykosiden, die die Komponente (a) bilden, stellen bekannte nichtionische Tenside dar, die der Formel (I) folgen,

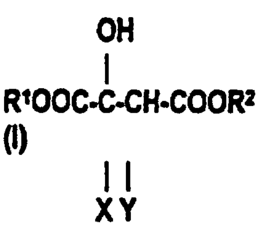

(I)

in der X für Wasserstoff oder eine $CH_2COOR^3$-Gruppe, Y für Wasserstoff oder eine Hydroxylgruppe, $R^1$, $R^2$ und $R^3$ unabhängig voneinander für Wasserstoff, ein Alkali- oder Erdalkalimetall, Ammonium, Alkyl-ammonium, Hydroxyalkylammonium, Glucammonium oder den Rest eines Alkylund/oder Alkenyloligoglykosids der Formel (II) steht,

$$R^4O\text{-}[G]_p$$
**(II)**

in der $R^4$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, mit den Maßgaben, daß für den Fall, daß X für eine $-CH_2COOR^3$-Gruppe steht, Y Wasserstoff bedeutet und wenigstens einer der Reste $R^1$, $R^2$ und $R^3$ für einen Glykosidrest steht. Die Herstellung und Verwendung dieser Stoffe als schaumstarke Tenside in kosmetischen Zubereitungen ist aus der europäischen Patentschrift **EP 0258814 B1** (Auschem) bekannt, auf die hier in vollem Umfang Bezug genommen werden soll.

Die Säurekomponente der nichtionischen Tenside kann sich von Äpfelsäure, Weinsäure oder Citronensäure sowie deren Gemischen ableiten. In Abhängigkeit der Zahl vorhandener Hydroxylgruppen in der Säurekomponente können Mono-, Di- oder Triester sowie deren technische Gemische vorliegen. Vorzugsweise gelangen jedoch Monoester bzw. Mischungen mit hohem Monoestergehalt zum Einsatz, welche vom Fachmann gemäß der Lehre der schon erwähnten EP 0258814 B1 erhalten werden können.

Die Alkyl- und/oder Alkenyloligoglykosidkomponente kann sich von Aldosen bzw. Ketosen mit 5 oder 6 Kohlenstoffatomen, vorzugsweise der Glucose ableiten. Die bevorzugten nichtionischen Tenside sind somit Ester von Alkyl- und/oder Alkenyloligoglucosiden. Die Indexzahl p in der allgemeinen Formel (II) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Ester eingesetzt, deren Alkyl- - und/oder Alkenyloligoglykosidkomponente einen mittleren Oligomerisierungsgrad p von 1,1 bis 3,0 aufweist. Aus anwendungstechnischer Sicht sind solche Alkyl- und/oder Alkenyloligoglykosidester bevorzugt, deren Oligomerisierungsgrad kleiner als 1,7 ist und insbesondere zwischen 1,2 und 1,4 liegt. Der Alkyl- bzw. Alkenylrest $R^1$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind $C_9$-$C_{11}$- bzw. $C_8$-$C_{10}$-Alkyloligoglucosidester mit einem DP = 1 bis 3, deren Alkylrest sich von entsprechenden Oxoalkoholen oder Alkoholen ableitet, die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können. Der Alkyl- bzw. Alkenylrest $R^1$ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucosidester auf Basis von gehärtetem $C_{12/14}$-Kokosalkohol mit einem DP von 1 bis 3. Entsprechende Produkte sind unter der Marke Eucarol® im Handel erhältlich.
Die Erfindung schließt die Erkenntnis ein, daß Mischungen von Hydroxycarbonsäurealkylund/oder alkenylglykosidestern und Alkyl- und/oder Alkenyloligoglykosiden zusammen mit Fettalkoholen besonders stabile Emulsionen ergeben. Solche ternären Zubereitungen können im einfachsten Fall durch Vermischen der drei Bestandteile hergestellt werden. In einer besonderen Ausführungsform der Erfindung werden jedoch Fettalkohole zusammen mit Hydroxycarbonsäureglykosidestern eingesetzt, welche noch 1 bis 50, vorzugsweise 5 bis 25 und insbesondere 10 bis 15 Gew.-% unveresterte Glykoside enthalten.

<u>Fettalkohole</u>

Unter Fettalkoholen, die die Komponente (b) bilden, sind primäre aliphatische Alkohole der Formel (III) zu verstehen,

$$R^5OH \qquad \text{(III)}$$

in der $R^5$ für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Patm-, Palmkem- oder Talgfettalkohol. Vorzugsweise kommen solche Fettalkohole zum Einsatz, welche den gleichen Alkylrest wie die Alkylglucosidkomponente der Hydroxycarbonsäureester aufweisen, also beispielsweise Cetearylalkohol in Kombination mit Citronensäurecetearylglucosidester. Die Zubereitungen können die Komponenten (a) und (b) im Gewichtsverhältnis 10 : 90 bis 90 : 10, vorzugsweise 25 : 75 bis 75 : 25 und insbesondere 40 : 60 bis 60 : 40 enthalten. Der Anteil der Mischungen der Komponenten (a) und (b) an den Zubereitungen kann in Summe 0,5 bis 20, vorzugsweise 1 bis 15 und insbesondere 5 bis 10 Gew.-% - bezogen auf die Mittel betragen.

Co-Emulgatoren

In einer bevorzugten Ausführungsform der Erfindung können die Zubereitungen als fakultative Komponente (c) weitere anionische, nichtionische, kationische, amphotere und/oder zwitterionische Co-Emulgatoren enthalten, wobei deren Anteil - bezogen auf die Mittel - 0,5 bis 10, vorzugsweise 1 bis 8 und insbesondere 2 bis 5 Gew.-% betragen kann. Als Co-Emulgatoren kommen beispielsweise nichtionogene Tenside aus mindestens einer der folgenden Gruppen in Frage:

(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) $C_{12/18}$-Fettsäurernono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöi und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter $C_{6/22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbohat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind aus **DE 2024051 PS** als Rückfettungsmittel für kosmetische Zubereitungen bekannt.

$C_{8/18}$-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12/18}$-Acylsarcosin. Als anionische Emulgatoren seien insbesondere Alkyl(ether)sulfate, Acylglutamate, Proteinfettsäurekondensate und Monoglyceridsulfate genannt. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

## Gewerbliche Anwendbarkeit

Die erfindungsgemäßen Zubereitungen, die die speziellen Hydroxycarbonsäureester zusammen mit Fettalkoholen enthalten, besitzen ausgezeichnete selbstemulgierende und konsistenzgebende Eigenschaften. Ein weiterer Gegenstand der Erfindung betrifft daher ihre Verwendung zur Hersbellung von kosmetischen und/oder pharmazeutischen Zubereitungen, in denen sie in Mengen von 0,5 bis 20, vorzugsweise 1 bis 15 und insbesondere 5 bis 10 Gew.-% - bezogen auf die Mittel - enthalten sein können.

### Kosmetische und/oder pharmazeutische Zubereitungen

Die erfindungsgemäßen Emulgatorgemische können zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/Fett-Massen, Stiftpräparaten, Pudem oder Salben dienen. Diese Mittel können ferner als weitere Hilfs- und Zusatzstoffe milde Tenside, Ölkörper, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe, keimhemmende Mittel und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, $\alpha$-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen, Ester von verzweigten $C_6$-$C_{13}$-Carbonsäuren mit linearen $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, I-sostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von Hydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, Guerbetcarbonate, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäune-estern mit Polyolen, Siliconöle und/oder aliphatische bzw. naphthenische Kohlenwasserstoffe, wie z.B. wie Squalan, Squalen oder Dialkylcydohexane in Betracht.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als sekundäre **Konsistenzgeber** kommen Hydroxyfettalkohole, Partialglyceride, Fettsäuren oder Hy-droxyfettsäuren in Betracht. Geeignete **Verdickungsmittel** sind beispielsweise Aerosil-Typen (hydro-phile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäunaglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

**Geeignete kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere** und **nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosidund/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können **Metallsatze** von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren,

Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc. Cosm.Chem. 24, 281 (1973)**]. Unter der Marke Locron® der Hoechst AG, Frankfurt/FRG, befindet beispielsweise sich ein Aluminiumchlorhydrat im Handel, das der Formel $[Al_2(OH)_5Cl]$ $*2,5\ H_2O$ entspricht und dessen Einsatz besonders bevorzugt ist [vgl. **J.Pharm.Pharmacol. 26, 531 (1975)**]. Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäure-diethylester, Adipinsäure, Adipinsäuremonoethylester, Adipinsäunrdiethylester, Malonsäure und Malonsäurediethylester, Hydroxycarbnonsäuren und deren Ester wie beispielsweise Citronen-säure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als Antischuppenmittel können Climbazol, Octopirox und Zinkpyrethion eingesetzt werden. Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:

- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Suffonsäurederivate von Benzophenonen, vorzugsweise 2-Hydroxy-4-methoxybenzophenon-5-sulfonsäure und ihre Salze;
- Sulfonsäunrderivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D, L-Camosin, D-Camosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothiogtucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, A-myl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycot sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insekten-Repellentien kommen N,N-Diethyi-m-toluamid, 1,2-Pentandiol oder Insekten-Repellent 3535 in Frage, als Selbstbräuner eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedem-, Rosenholz), Kräutem und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat. Phenylethylacetat, Linalylbenzoat, Benzylformiat, Ethylmethylphenylglycinat, Allylcyclohexylpropionat, Styrallylpropionat und Benrylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilliat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Famesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den auf den Feststoffanteil der Zubereitungen.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

## Beispiele

Unter Einsatz von Coco Glycerides (Myritol® 331, Henkel KGaA) als Ölkomponente und verschiedenen Emulgatoren wurden nach dem PIT-Verfahren Emulsionen hergestellt und in einem Brookfield RVT-Viskosimeter auf ihre Anfangsviskosität (20°C, 4 UpM, Spindel TE, mit Helipath) untersucht. Anschließend wurde die Stabilität der Emulsionen bei Lagerung über einen Zeitraum von 1 bis 4 Wochen bei 20 bzw. 40°C überprüft. Dabei bedeutet (+) stabil und (-) Phasentrennung. Die Ergebnisse sind in Tabelle 1 zusammengefaßt. Die Beispiele 1 bis 6 sind erfindungsgemäß, die Beispiele V1 bis V4 dienen zum Vergleich.

**Tabelle 1**
**Viskosität und Emulsionsstabilität**

| Zusammensetzung | 1 | 2 | 3 | 4 | 5 | 6 | V1 | V2 | V3 | V4 |
|---|---|---|---|---|---|---|---|---|---|---|
| Sodium Laurylglucoside Citrate | 1,5 | 1,0 | 0,5 | - | - | 1,0 | 2,0 | - | - | - |
| Sodium Laurylglucoside Malate | - | - | - | 1,0 | - | - | - | - | - | - |
| Sodium Cetearylglucoside Tartrate | - | - | - | - | 1,0 | - | - | - | - | - |
| Lauryl Glucoside | - | - | - | - | - | 1,0 | - | - | - | - |
| Cetearyl Glucoside | - | - | - | - | - | - | - | - | 2,0 | 1,0 |
| Coco Glycerides | 15,0 | 15,0 | 15,0 | 15,0 | 15,0 | 15,0 | 15,0 | 15,0 | 15,0 | 15,0 |
| Cetearyl Alcohol | 0,5 | 1,0 | 1,5 | 1,0 | 1,0 | 1,0 | - | 2,0 | - | 1,0 |
| Wasser | ad 100 | | | | | | | | | |
| ***Viskosität [Pas]*** | 420 | 450 | 420 | 440 | 500 | 480 | 120 | 400 | 80 | 370 |
| ***Stabilität*** | | | | | | | | | | |
| - nach 1 Woche, 20°C | + | + | + | + | + | + | + | + | + | + |
| - nach 1 Woche, 40°C | + | + | + | + | + | + | - | - | - | + |
| - nach 4 Wochen, 40°C | + | + | + | + | + | + | - | - | - | - |

In der nachfolgenden Tabelle befinden sich zahlreiche Formulierungsbeispiele. Alle Mengenangaben verstehen sich dabei als Gew.-%.

**Tabelle 2**
**Kosmetische Zubereitungen (Wasser, Konservierungsmittel ad 100 Gew.-%)**

| Zusammensetzung (INCI) | 1 | 2 | 3 | 4 | 5 | 6 | 7 | 8 | 9 | 10 |
|---|---|---|---|---|---|---|---|---|---|---|
| **Texapon® NSO**<br>Sodium Laureth Sulfate | | - | - | - | - | - | 38,0 | 38,0 | 35,0 | |
| **Plantacare® 818**<br>Coco Glucosides | 2,0 | - | - | - | - | - | 7,0 | 7,0 | 6,0 | - |
| **Plantacare® PS 10**<br>Sodium Laureth Sulfate (and) Coco Glucosides | - | - | - | - | - | - | - | - | - | 16,0 |
| **Dehyquart AU 56**<br>Dipalmoylethyl Hydroxyethylmonium Methosulfate | 1,0 | 1,0 | 1,0 | 1,0 | 1,0 | 1,0 | - | - | - | - |
| **Dehyton® PK 45**<br>Cocamidopropyl Betaine | - | - | - | - | - | - | - | - | 10,0 | |
| **Sodium Laurylglucoside Citrate** | 3,0 | - | - | 1,0 | - | - | 1.0 | - | - | 1,0 |
| **Sodium Laurylglucoside Malate** | - | 1,0 | - | - | 1,0 | - | - | 1,0 | - | - |
| **Sodium Cetearylglucoside Tartrate** | - | - | 1,0 | - | - | 1,0 | - | - | 1,0 | - |
| **Emulgade® PL 68/50**<br>Cetearyl Glucoside (and) Cetearyl Alcohol | 3,0 | - | - | - | - | - | - | - | - | - |
| **Eumulgin® B2**<br>Ceteareth-20 | - | 0,8 | - | 0,8 | - | 1,0 | - | - | - | - |
| **Eumulgin® VL 75**<br>Lauryl Glucoside (and) Polyglyceryl-2 Polyhydroxystearate (and) Glycerin | 3,0 | - | 0,8 | - | 0.8 | - | - | - | - | - |
| **Lanette® O**<br>Cetearyl Alcohol | 1,0 | 2,5 | 2,5 | 2,5 | 3,0 | 2,5 | 1,0 | 1,0 | 1,0 | 1,0 |
| **Lameform® TGI**<br>Polyglyceryl-3 Isostearate | 1,0 | - | - | 1,0 | - | - | - | - | - | - |
| **Dehymuls® PGPH**<br>Polyglyceryl-2 Dipolyhydroxystearate | - | - | - | - | 1,0 | - | - | - | - | - |
| **Cutina® GMS**<br>Glyceryl Stearate | 0,5 | 0,5 | 0,5 | 0,5 | 0,5 | 1,0 | - | - | - | - |
| **Cetiol® HE**<br>PEG-7 Glyceryl Cocoate | - | - | - | - | - | - | - | - | 1,0 | |
| **Cetiol® OE**<br>Dicaprylyl Ether | 1.0 | 1,0 | 1,0 | - | - | - | - | - | - | - |
| **Cetiol® PGL**<br>Hexyldecanol (and) Hexyldecyl Laurate | - | - | - | - | 1,0 | - | - | - | - | - |
| **Eutanol® G**<br>Octyldodecanol | - | - | 1,0 | - | - | 1,0 | - | - | - | - |
| **Nutrilan® Keratin W**<br>Hydrolyzed Keratin | 2,3 | - | - | 2,0 | - | - | - | - | - | - |
| **Lamesoft® LMG**<br>Glyceryl Laurate (and) Potassium Cocoyl Hydrolyzed Collagen | - | - | - | - | - | - | 3,0 | 2,0 | 4,0 | |
| **Gluadin® WK**<br>Sodium Cocoyl Hydrolyzed Wheat Protein | 1,0 | 1,0 | 1,0 | 1,0 | 1,0 | 1,5 | 0,5 | 0,5 | 0,5 | 0,5 |
| **Euperlan® PK 3000 AM**<br>Glycol Distearate (and) Laureth-4 (and) Cocamidopropyl Betaine | - | - | - | - | - | - | - | 3,0 | 5,0 | 5,0 |
| **Generol® 122 N**<br>Soja Sterol | - | - | - | - | 1,0 | 1,0 | - | - | - | - |
| **Copherol® 12250**<br>Tocopherol Acetate | - | - | 0,1 | 0,1 | - | - | - | - | - | - |
| **Arlypon® F**<br>Laureth-2 | | | | | | | 3,0 | 3,0 | 1,0 | |
| **Sodium Chloride** | - | - | - | - | - | - | - | 1.5 | | 1,5 |

(1-4) Haarspülung, (5-6) Haarkur, (7-8) Duschbad, (9) Duschgel, (10) Waschlotion

Tabelle 2
Kosmetische Zubereitungen (Wasser, Konservierungsmittel ad 100 Gew.-%) - Fortsetzung 1

| Zusammensetzung (INCI) | 11 | 12 | 13 | 14 | 15 | 16 | 17 | 18 | 19 | 20 |
|---|---|---|---|---|---|---|---|---|---|---|
| Texapon® NSO<br>Sodium Laureth Sulfate | 20,0 | 20,0 | 12,4 | - | 25,0 | 11,0 | - | - | - | - |
| Texapon® K 14 S<br>Sodium Myreth Sulfate | - | - | - | - | - | - | - | - | 11,0 | 23,0 |
| Texapon® SB 3<br>Disodium Laureth Sulfosuccinate | - | - | - | - | - | 7,0 | - | - | - | - |
| Plantacare® 818<br>Coco Glucosides | 5,0 | 5,0 | 4,0 | - | - | - | - | - | 6,0 | 4,0 |
| Plantacare® 2000<br>Decyl Glucoside | - | - | - | - | 5,0 | 4,0 | - | - | - | - |
| Plantacare® PS 10<br>Sodium Laureth Sulfate (and) Coco Glucosides | - | - | - | 40,0 | - | - | 16,0 | 17,0 | - | - |
| Sodium Laurylglucoside Citrate | 1,0 | - | - | 1,0 | - | - | 1,0 | - | - | 1,0 |
| Sodium Laurylglucoside Malate | - | 1,0 | - | - | 1,0 | - | - | 1,0 | - | - |
| Sodium Cetearylglucoside Tartrate | - | - | 1,0 | - | - | 1,0 | - | - | 1,0 | - |
| Dehyton® PK 45<br>Cocamidopropyl Betaine | 20,0 | 20,0 | - | - | 8,0 | - | - | - | - | 7,0 |
| Emulgade® PL 68/50<br>Cetearyl Glucoside (and) Cetearyl Alcohol | - | - | - | - | - | - | - | - | - | - |
| Eumulgin® B1<br>Ceteareth-12 | - | - | - | - | 1,0 | - | - | - | - | - |
| Eumulgin® B2<br>Ceteareth-20 | - | - | - | 1,0 | - | - | - | - | - | - |
| Lanette® O<br>Cetearyl Alcohol | 0,5 | 0,5 | 0,5 | 0,5 | 0,5 | 0,5 | 0,5 | 0,5 | 0,5 | 0,5 |
| Lameform® TGI<br>Polyglyceryl-3 Isostearate | - | - | - | 4,0 | - | - | - | - | - | - |
| Dehymuls® PGPH<br>Polyglyceryl-2 Dipolyhydroxystearate | - | - | 1,0 | - | - | - | - | - | - | - |
| Monomuls® 90-L 12<br>Glyceryl Laurate | - | - | - | - | - | - | - | - | - | 1,0 |
| Cutina® GMS<br>Glyceryl Stearate | - | - | - | - | - | - | - | - | 1,0 | - |
| Cetiol® HE<br>PEG-7 Glyceryl Cocoate | - | 0,2 | - | - | - | - | - | - | - | - |
| Eutanol® G<br>Octyldodecanol | - | - | - | 3,0 | - | - | - | - | - | - |
| Nutrilan® Keratin W<br>Hydrolyzed Keratin | - | - | - | - | - | - | - | - | 2,0 | 2,0 |
| Nutrilan® I<br>Hydrolyzed Collagen | 1,0 | - | - | - | - | 2,0 | - | 2,0 | 1,0 | 5,0 |
| Gluadin® WK<br>Sodium Cocoyl Hydrolyzed Wheat Protein | 1,0 | 1,5 | 4,0 | 1,0 | 3,0 | 1,0 | 2,0 | 2,0 | 2,0 | - |
| Euperlan® PK 3000 AM<br>Glycol Distearate (and) Laureth-4 (and) Cocamidopropyl Betaine | 5,0 | 3,0 | 4,0 | - | - | - | - | 3,0 | 3,0 | - |
| Panthenol | - | - | 1,0 | - | - | - | - | - | - | - |
| Arlypon® F<br>Laureth-2 | 2,6 | 1,6 | - | 1,0 | 1,5 | - | - | - | - | - |
| Sodium Chloride | - | - | - | - | - | 1,6 | 2,0 | 2,2 | - | 3,0 |
| Glycerin (86 Gew.-%ig) | - | 5,0 | - | - | - | - | - | 1,0 | 3,0 | - |

(11-14) Duschbad „Two-in-One), (15-20) Shampoo

**Tabelle 2**
**Kosmetische Zubereitungen (Wasser, Konservierungsmittel ad 100 Gew.-%) - Fortsetzung 2**

| Zusammensetzung (INCI) | 21 | 22 | 23 | 24 | 25 | 26 | 27 | 28 | 29 | 30 |
|---|---|---|---|---|---|---|---|---|---|---|
| **Texapon® NSO**<br>Sodium Laureth Sulfate | - | 30,0 | - | - | 25,0 | - | - | - | - | - |
| **Plantacare® 818**<br>Coco Glucosides | - | 10,0 | 30,0 | - | 20,0 | - | - | - | - | - |
| **Plantacare® PS 10**<br>Sodium Laureth Sulfate (and) Coco Glucosides | 22,0 | - | - | 22,0 | - | - | - | - | - | - |
| **Sodium Laurylglucoside Citrate** | 1,0 | - | - | 1,0 | - | - | 1.0 | - | - | 1,0 |
| **Sodium Laurylglucoside Malate** | - | 1,0 | - | - | 1,0 | - | - | 1,0 | - | - |
| **Sodium Cetearylglucoside Tartrate** | - | - | 1,0 | - | - | 1,0 | - | - | 1,0 | - |
| **Dehyton® PK 45**<br>Cocamidopropyl Betaine | 15,0 | 10,0 | 10,0 | 15,0 | 20,0 | - | - | - | - | - |
| **Eumulgin® B1**<br>Ceteareth-12 | - | - | 15,0 | - | - | - | - | - | - | - |
| **Eumulgin® HRE 60**<br>PEG 60 Hydrogenated Castor Oil | - | - | - | - | 5,0 | - | - | - | - | 4,0 |
| **Lameform® TGI**<br>Polyglyceryl-3 Isostearate | - | - | - | - | - | - | - | - | 4,0 | - |
| **Dehymuls® PGPH**<br>Polyglyceryl-2 Dipolyhydroxystearate | - | - | 3,8 | - | - | - | - | - | - | - |
| **Lanette® O**<br>Cetearyl Alcohol | 1,0 | 1,0 | 1,0 | 1,0 | 1,0 | 1,0 | 1,0 | 1,0 | 1,0 | 1,0 |
| **Monomuls® 90-O 18**<br>Glyceryl Oleate | - | - | - | - | - | - | - | | 2,0 | - |
| **Cetiol® HE**<br>PEG-7 Glyceryl Cocoate | 2,0 | - | - | 2,0 | 5,0 | - | - | - | - | - |
| **Cetiol® OE**<br>Dicaprylyl Ether | - | - | - | - | - | - | - | - | 5,0 | 7,0 |
| **Cetiol® SN**<br>Cetearyl Isononanoate | - | - | - | - | - | 6,0 | 6,0 | - | 10,0 | 8,0 |
| **Myritol® 318**<br>Coco Caprylate Caprate | - | - | - | - | - | - | - | 6,0 | 5,0 | 6,0 |
| **Melissenöl** | - | - | 5,0 | - | - | - | - | - | - | - |
| **Bees Wax** | - | - | - | - | - | - | - | - | 7,0 | 5,0 |
| **Nutrilan® I**<br>Hydrolyzed Collagen | - | - | - | - | 2,0 | 4,0 | 6,0 | - | - | - |
| **Lamesoft® LMG**<br>Glyceryl Laurate (and) Potassium Cocoyl Hydrolyzed Collagen | - | 4,0 | - | - | - | - | - | 6,0 | - | - |
| **Gluadin® AGP**<br>Hydrolyzed Wheat Gluten | 0,5 | 0,5 | - | - | - | - | - | - | - | 5,0 |
| **Gluadin® WK**<br>Sodium Cocoyl Hydrolyzed Wheat Protein | 2,0 | 2,0 | 4,0 | 2,0 | 5,0 | - | - | - | 5,0 | - |
| **Euperlan® PK 3000 AM**<br>Glycol Distearate (and) Laureth-4 (and) Cocamidopropyl Betaine | 5,0 | - | - | 5,0 | - | - | - | - | - | - |
| **Arlypon® F**<br>Laureth-2 | - | - | 1,5 | - | - | - | - | - | - | - |
| **Magnesium Sulfate Hepta Hydrate** | - | - | - | - | - | - | - | - | 1,0 | 1,0 |
| **Glycerin (86 Gew.-%ig)** | - | - | - | - | - | 3,0 | 3,0 | 3,0 | 5,0 | 5,0 |

(21-25) Schaumbad, (26) Softcreme, (27, 28) Feuchtigkeitsemulsion, (29, 30) Nachtcreme

**Tabelle 2**
**Kosmetische Zubereitungen (Wasser, Konservierungsmittel ad 100 Gew.-%) - Fortsetzung 3**

| Zusammensetzung (INCI) | 31 | 32 | 33 | 34 | 35 | 36 | 37 | 38 | 39 | 40 |
|---|---|---|---|---|---|---|---|---|---|---|
| **Dehymuls® PGPH** Polyglyceryl-2 Dipolyhydroxystearate | 2,0 | 3,0 | - | 5,0 | - | - | - | - | - | - |
| **Lameform® TGI** Polyglyceryl-3 Diisostearate | 4,0 | 1,0 | - | - | - | - | - | - | - | - |
| **Abil® EM 90** Cetyl Dimethicone Copolyol | - | - | 3,0 | - | - | - | - | - | - | - |
| **Sodium Laurylglucoside Citrate** | 1,0 | - | - | 1,0 | - | - | 1,0 | - | - | 1,0 |
| **Sodium Laurylglucoside Malate** | - | 1,0 | - | - | 1,0 | - | - | 1,0 | - | - |
| **Sodium Cetearylglucoside Tartrate** | - | - | 1,0 | - | - | 1,0 | - | - | 1,0 | - |
| **Emulgade® PL 68/50** Cetearyl Glucoside (and) Cetearyl Alcohol | - | - | - | - | 4,0 | - | - | 2,0 | 3,0 | - |
| **Eumulgin VL 75** Polyglyceryl-2 Dipolyhydroxystearate (and) Lauryl Glucoside (and) Glycerin | - | - | - | - | - | 3,5 | 4,0 | - | 2,5 | - |
| **Bees Wax** | 3,0 | 2,0 | 5,0 | 2,0 | - | - | - | - | - | - |
| **Cutina® GMS** Glyceryl Stearate | - | - | - | - | - | 2,0 | 4,0 | - | - | 4,0 |
| **Lanette® O** Cetearyl Alcohol | 1,0 | 1,0 | 2,0 | 1,0 | 2,0 | 4,0 | 2,0 | 4,0 | 4,0 | 1,0 |
| **Antaron® V 216** PVP / Hexadecene Copolymer | - | - | - | - | - | 3,0 | - | - | - | 2,0 |
| **Plantaren® 818** Cocoglycerides | 5,0 | 6,0 | 10,0 | 2,0 | 8,0 | 6,0 | 6,0 | - | 5,0 | 5,0 |
| **Dioctyl Carbonate** | 5,0 | 4,0 | 6,0 | 8,0 | 6,0 | 5,0 | 4,0 | 3,0 | 4,0 | 6,0 |
| **Cetiol® J 600** Oleyl Erucate | 2,0 | - | 3,0 | 5,0 | 4,0 | 3,0 | 3,0 | - | 5,0 | 4,0 |
| **Cetiol® OE** Dicaprylyl Ether | 3,0 | - | - | - | - | 1,0 | - | - | - | - |
| **Mineral Oil** | - | 4,0 | - | 4,0 | - | 2,0 | - | 1,0 | - | - |
| **Cetiol® PGL** Hexadecanol (and) Hexyldecyl Laurate | - | 7,0 | 3,0 | 7,0 | 4,0 | - | - | - | 1,0 | - |
| **Panthenol / Bisabolol** | 1,2 | 1,2 | 1,2 | 1,2 | 1,2 | 1,2 | 1,2 | 1,2 | 1,2 | 1,2 |
| **Copherol® F 1300** Tocopherol / Tocopheyl Acetate | 0,5 | 1,0 | 1,0 | 2,0 | 1,0 | 1,0 | 1,0 | 2,0 | 0,5 | 2,0 |
| **Neo Heliopan® Hydro** Sodium Phenylbenzimidazole Sulfonate | 3,0 | - | - | 3,0 | - | - | 2,0 | - | 2,0 | - |
| **Neo Heliopan® 303** Octocrylene | - | 5,0 | - | - | - | 4,0 | 5,0 | - | - | 10,0 |
| **Neo Heliopan® BB** Benzophenone-3 | 1,5 | - | - | 2,0 | 1,5 | - | - | - | 2,0 | - |
| **Neo Heliopan® E 1000** Isoamyl p-Methoxycinnamate | 5,0 | - | 4,0 | - | 2,0 | 2,0 | 4,0 | 10,0 | - | - |
| **Neo Heliopan® AV** Octyl Methoxycinnamate | 4,0 | - | 4,0 | 3,0 | 2,0 | 3,0 | 4,0 | - | 10,0 | 2,0 |
| **Uvinul® T 150** Octyl triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | - | - | - | - | 5,0 |
| **Titanium Dioxide** | - | 2,0 | 2,0 | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |

(41) W/O-Sonnenschutzcreme, (42-44) W/O-Sonnenschutzlotion, (45, 48, 50) O/W-Sonnenschutzlotion (46, 47, 49) O/W-Sonnenschutzcreme

**Patentansprüche**

1. Kosmetische und/oder pharmazeutische Zubereitungen, enthaltend

   (a) Hydroxycarbonsäurealkyl- und/oder -alkenyloligoglykosidester und
   (b) Fettalkohole.

2. Zubereitungen nach Anspruch 1, **dadurch gekennzeichnet, daß** sie als Komponente (a) Hydroxy-carbonsäurealkyl- und/oder -alkenyloligoglykosidester der Formel (I) enthalten,

   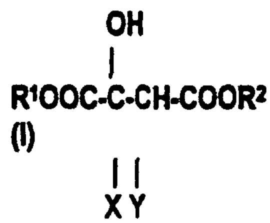
   (I)

   in der X für Wasserstoff oder eine $CH_2COOR^3$-Gruppe, Y für Wasserstoff oder eine Hydroxylgruppe, $R^1$, $R^2$ und $R^3$ unabhängig voneinander für Wasserstoff, ein Alkali- oder Erdalkalimetall, Ammonium, Alkylammonium, Hydroxyalkylammonium, Glucammonium oder den Rest eines Alkyl- und/oder Alkenyloligoglykosids der Formel (II) steht,

   $$R^4O\text{-}[G]_p \quad \text{(II)}$$

   in der $R^4$ für einen Alkyl- und/oder Alkenylrest mit 4 bis 22 Kohlenstoffatomen, G für einen Zucker-rest mit 5 oder 6 Kohlenstoffatomen und p für Zahlen von 1 bis 10 steht, mit den Maßgaben, daß für den Fall, daß X für eine $CH_2COOR^3$-Gruppe steht, Y Wasserstoff bedeutet und wenigstens einer der Reste $R^1$, $R^2$ und $R^3$ für einen Glykosidrest steht.

3. Zubereitungen nach den Ansprüchen 1 und/oder 2, **dadurch gekennzeichnet, daß** sie Ester von Äpfelsäure, Weinsäure und/oder Citronensäure mit Alkyl- und/oder Alkenyloligoglykosiden enthalten.

4. Zubereitungen nach mindestens einem der Ansprüche 1 bis 3, **dadurch gekennzeichnet, daß** sie Ester enthalten, welche noch 1 bis 50 Gew.-% unveresterte Glykoside enthalten.

5. Zubereitungen nach mindestens einem der Ansprüche 1 bis 4, **dadurch gekennzeichnet, daß** sie Fettalkohole der Formel (III) enthalten,

   $$R^5OH \quad \text{(III)}$$

   in der $R^5$ für einen aliphatischen, linearen oder verzweigten Kohlenwasserstoffrest mit 6 bis 22 Kohlenstoffatomen und 0 und/oder 1, 2 oder 3 Doppelbindungen steht.

6. Zubereitungen nach mindestens einem der Ansprüche 1 bis 5, **dadurch gekennzeichnet, daß** sie als Komponente (b) Fettalkohole enthalten, deren Alkylreste mit den Alkylresten der Alkylglykosidkomponente der Hydroxycarbonsäureester übereinstimmen.

7. Zubereitungen nach mindestens einem der Ansprüche 1 bis 6, **dadurch gekennzeichnet, daß** sie die Komponenten (a) und (b) im Gewichtsverhältnis 10: 90 bis 90 : 10 enthalten.

8. Zubereitungen nach mindestens einem der Ansprüche 1 bis 7, **dadurch gekennzeichnet, daß** sie die Mischungen der Komponenten (a) und (b) in Summe in Mengen von 0,5 bis 20 Gew.-% - bezogen auf die Mittel - enthalten.

9. Zubereitungen nach mindestens einem der Ansprüche 1 bis 8, **dadurch gekennzeichnet, daß** sie als fakultative Komponente (c) anionische, nichtionische, kationische, amphotere und/oder zwitterionische Co-Emulgatoren enthalten.

**10.** Verwendung von Mischungen, enthaltend

(a) Hydroxycarbonsäurealkyl- und/oder -alkenyloligoglykosidester und
(b) Fettalkohole

zur Herstellung von kosmetischen und/oder pharmazeutischen Zubereitungen.

## Claims

**1.** Cosmetic and/or pharmaceutical preparations containing

(a) hydroxycarboxylic acid alkyl and/or alkenyl oligoglycoside esters and
(b) fatty alcohols.

**2.** Preparations as claimed in claim 1, **characterized in that** they contain as component (a) hydroxycarboxylic acid and alkyl and/or alkenyl oligoglycoside esters corresponding to formula (I):

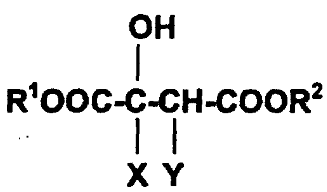

**(I)**

where X is hydrogen or a $CH_2COOR^3$ group, Y is hydrogen or a hydroxyl group, $R^1$, $R^2$ and $R^3$ independently of one another represent hydrogen, an alkali metal or alkaline earth metal, ammonium, alkyl ammonium, hydroxyalkyl ammonium, glucammonium or the residue of an alkyl and/or alkenyl oligoglycoside corresponding to formula (II):

$$R^4O\text{-}[G]_p \qquad \text{(II)}$$

in which $R^4$ is an alkyl and/or alkenyl group containing 4 to 22 carbon atoms, G is a sugar unit containing 5 or 6 carbon atoms and p is a number of 1 to 10, with the provisos that, where X is a $CH_2COOR^3$ group, Y is hydrogen and at least one of the substituents $R^1$, $R^2$ and $R^3$ is a glycoside unit.

**3.** Preparations as claimed in claims 1 and/or 2, **characterized in that** they contain esters of malic acid, tartaric acid and/or citric acid with alkyl and/or alkenyl oligoglycosides.

**4.** Preparations as claimed in at least one of claims 1 to 3, **characterized in that** they contain esters which still contain 1 to 50% by weight of unesterified glycosides.

**5.** Preparations as claimed in at least one of claims 1 to 4, **characterized in that** they contain fatty alcohols corresponding to formula **(III):**

$$R^5OH \text{ (III)}$$

in which $R^5$ is an aliphatic, linear or branched hydrocarbon radical containing 6 to 22 carbon atoms and 0 and/or 1, 2 or 3 double bonds.

**6.** Preparations as claimed in at least one of claims 1 to 5, **characterized in that** they contain as component (b) fatty alcohols of which the alkyl groups correspond to the alkyl groups of the alkyl glycoside component of the hydroxycarboxylic acid esters.

7. Preparations as claimed in at least one of claims 1 to 6, **characterized in that** they contain components (a) and (b) in a ratio by weight of 10:90 to 90:10.

8. Preparations as claimed in at least one of claims 1 to 7, **characterized in that** they contain the mixtures of components (a) and (b) in total quantities of 0.5 to 20% by weight, based on the preparation.

9. Preparations as claimed in at least one of claims 1 to 8, **characterized in that** they contain anionic, nonionic, cationic, amphoteric and/or zwitterionic co-emulsifiers as an optional component (c).

10. The use of mixtures containing

(a) hydroxycarboxylic acid alkyl and/or alkenyl oligoglycoside esters and
(b) fatty alcohols

for the production of cosmetic and/or pharmaceutical preparations.

**Revendications**

1. Préparations cosmétiques et/ou pharmaceutiques contenant

(a) des esters d'alkyl et/ou d'alkényloligoglycoside d'acide hydroxycarboxylique et
(b) des alcools gras.

2. Préparations selon la revendication 1,
**caractérisées en ce qu'**
elles renferment comme composant (a) des esters d'alkyl et/ou d'alkényloligoglycoside d'acide hydroxycarboxylique de formule (I)

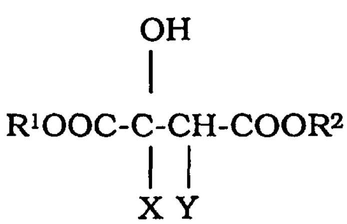

(I)

dans laquelle X représente de l'hydrogène ou un groupe $CH_2COOR^3$, Y représente de l'hydrogène ou un groupe hydroxyle, $R^1$, $R^2$ et $R^3$ indépendamment les uns des autres, représentent de l'hydrogène, un métal alcalin ou alcalinoterreux, un ammonium, un alkylammonium, un hydroxyalkylammonium, un glucammonium ou le reste d'un alkyl- et/ou alkényloligoglycoside de formule (II)

$$R^4O\text{-}[G]_p \qquad \text{(II)}$$

dans laquelle $R^4$ représente un reste alkyle et/ou alkenyle ayant de 4 à 22 atomes de carbone, G représente un reste de sucre ayant 5 ou 6 atomes de carbone et p représente des nombres allant de 1 à 10, avec la limitation que pour le cas où X représente un groupe $-CH_2COOR^3$, Y signifie de l'hydrogène et au moins un des restes $R^1$, $R^2$ et $R^3$ représente un reste glycoside.

3. Préparations selon les revendications 1 et/ou 2,
**caractérisées en ce qu'**
elles renferment des esters d'acide malique, d'acide tartrique et/ou d'acide citrique avec des alkyl- et/ou alkényloligoglycosides.

4. Préparations selon au moins l'une des revendications 1 à 3,
**caractérisées en ce qu'**
elles renferment des esters qui contiennent encore de 1 à 50 % en poids de glycosides non estérifiés.

5. Préparations selon au moins l'une des revendications 1 à 4,
**caractérisées en ce qu'**
elles renferment des alcools gras de formule (III)

$$R^5OH \quad \text{(III)}$$

dans laquelle R5 représente un reste hydrocarboné aliphatique, linéaire ou ramifié, ayant de 6 à 22 atomes de carbone et 0 et/ou 1, 2 ou 3 doubles liaisons.

6. Préparations selon au moins l'une des revendications 1 à 5,
**caractérisées en ce qu'**
elles renferment comme composant (b) des alcools gras dont les restes alkyle sont en accord avec les restes alkyle du composant alkyloligoglycoside des esters d'acide hydroxycarboxylique.

7. Préparations selon au moins l'une des revendications 1 à 6,
**caractérisées en ce qu'**
elles renferment les composants (a) et (b) dans le rapport en poids de 10 : 90 à 90 : 10.

8. Préparations selon au moins l'une des revendications 1 à 7,
**caractérisées en ce qu'**
elles renferment les mélanges des composants (a) et (b) globalement en quantités allant de 0,5 à 20 % en poids rapporté à la composition.

9. Préparations selon au moins l'une des revendications 1 à 8,
**caractérisées en ce qu'**
elles renferment comme composant facultatif (c), des agents co-émulsionnants amioniques, non ioniques, cationiques, amphotères et/ou zwitterioniques.

10. Utilisation de mélanges renfermant :

(a) des esters d'alkyl- et/ou alkényloligoglycoside d'acide hydroxycarboxylique et
(b) des alcools gras

en vue de la production de préparations cosmétiques et/ou pharmaceutiques.